# EUROPEAN PATENT APPLICATION

(11) **EP 3 798 301 A1**
(43) Date of publication of application: **31.03.2021**
(21) Application number: 19199614.9
(22) Date of filing: 25.09.2019
(51) Int. Cl.: C12N 9/22, C12N 15/10, C12N 15/82

(54) **PROMOTER REPRESSION**

(71) Applicant: KWS SAAT SE & Co. KGaA, 37574 Einbeck (DE)
(72) Inventor: STREITNER, Corinna, 49328 Melle (DE); WELTMEIER, Fridtjof, 37574 Einbeck (DE)
(74) Representative: Eisenführ Speiser

(57) **Abstract**

The present invention relates to novel strategies for gradually decreasing the expression of genes by modifying the promoter sequence. Provided are methods for gradually decreasing the expression level of a nucleic acid molecule of interest in a cell, preferably in a plant cell, comprising the step of introducing at least one modification into a promoter sequence, wherein the modification disrupts a core promoter consensus sequence. Furthermore, the invention also provides methods for producing a cell or an organism, preferably a plant cell or a plant, having a decreased expression level of a nucleic acid molecule of interest. The invention also relates to a cell and an organism, preferably a plant cell and a plant, obtained by a method according to the invention.

SEQUENCE LISTING

<110>  KWS SAAT SE & Co. KGaA

<120>  Promoter repression

<130>  KWS354EP

<160>  38

<170>  PatentIn version 3.5

<210>  1
<211>  314
<212>  DNA
<213>  Zea mays

<400>  1
gatccgaccc ggcgtgctct acatatgcgt cccgtcaccc ctctcctctt catcctctct      60

tttcttcgcc caaatctcgc gtgctgcctc cgtcctgccc ccagcgcgtg cgctccgatc     120

cgccgggttt cagcgctgcg gctgtcgatt tacgcgattt gtctggcaga atacagttca     180

gagcctcgag cgctgcagat cgaggacctg gtcgtcggat tcgggcgccg cgggtgagac     240

ctaggcgccg gagtttttgt gacggagttt tggcgtggtt ggtggagggg gggaggaagg     300

agcggtctca tatg                                                       314


<210>  2
<211>  314
<212>  DNA
<213>  Artificial Sequence

<220>
<223>  ZmCPL3v3 (Figure 2) embedded in SEQ ID NO: 1

<400>  2
gatccgaccc ggcgtgctct acagatgcgt cccgtcaccc ctctcctctt catcctctct      60

tttcttcgcc caaatctcgc gtgctgcctc cgtcctgccc ccagcgcgtg cgctccgatc     120

cgccgggttt cagcgctgcg gctgtcgatt tacgcgattt gtctggcaga atacagttca     180

gagcctcgag cgctgcagat cgaggacctg gtcgtcggat tcgggcgccg cgggtgagac     240

ctaggcgccg gagtttttgt gacggagttt tggcgtggtt ggtggagggg gggaggaagg     300

agcggtctca tatg                                                       314


<210>  3
<211>  314
<212>  DNA
<213>  Artificial Sequence

<220>
<223>  ZmCPL3v4 (Figure 2) embedded in SEQ ID NO: 1

<400>  3
gatccgaccc ggcgtgctca acatatgcgt cccgtcaccc ctctcctctt catcctctct      60

tttcttcgcc caaatctcgc gtgctgcctc cgtcctgccc ccagcgcgtg cgctccgatc 120

cgccgggttt cagcgctgcg gctgtcgatt tacgcgattt gtctggcaga atacagttca 180

gagcctcgag cgctgcagat cgaggacctg gtcgtcggat tcgggcgccg cgggtgagac 240

ctaggcgccg gagtttttgt gacggagttt tggcgtggtt ggtggagggg gggaggaagg 300

agcggtctca tatg 314

<210> 4
<211> 314
<212> DNA
<213> Artificial Sequence

<220>
<223> ZmCPL3v5 (Figure 2) embedded in SEQ ID NO: 1

<400> 4
gatccgaccc ggcgtgctct acatacccgt cccgtcaccc ctctcctctt catcctctct 60

tttcttcgcc caaatctcgc gtgctgcctc cgtcctgccc ccagcgcgtg cgctccgatc 120

cgccgggttt cagcgctgcg gctgtcgatt tacgcgattt gtctggcaga atacagttca 180

gagcctcgag cgctgcagat cgaggacctg gtcgtcggat tcgggcgccg cgggtgagac 240

ctaggcgccg gagtttttgt gacggagttt tggcgtggtt ggtggagggg gggaggaagg 300

agcggtctca tatg 314

<210> 5
<211> 314
<212> DNA
<213> Artificial Sequence

<220>
<223> ZmCPL3v6 (Figure 2) embedded in SEQ ID NO: 1

<400> 5
gatccgaccc ggcgtgcttt acatatgcgt cccgtcaccc ctctcctctt catcctctct 60

tttcttcgcc caaatctcgc gtgctgcctc cgtcctgccc ccagcgcgtg cgctccgatc 120

cgccgggttt cagcgctgcg gctgtcgatt tacgcgattt gtctggcaga atacagttca 180

gagcctcgag cgctgcagat cgaggacctg gtcgtcggat tcgggcgccg cgggtgagac 240

ctaggcgccg gagtttttgt gacggagttt tggcgtggtt ggtggagggg gggaggaagg 300

agcggtctca tatg 314

<210> 6
<211> 314
<212> DNA
<213> Artificial Sequence

<220>
<223> ZmCPL3v7 (Figure 2) embedded in SEQ ID NO: 1

<400>  6
gatccgaccc ggcgtgctct atatatgcgt cccgtcaccc ctctcctctt catcctctct      60

tttcttcgcc caaatctcgc gtgctgcctc cgtcctgccc ccagcgcgtg cgctccgatc      120

cgccgggttt cagcgctgcg gctgtcgatt tacgcgattt gtctggcaga atacagttca      180

gagcctcgag cgctgcagat cgaggacctg gtcgtcggat tcgggcgccg cgggtgagac      240

ctaggcgccg gagtttttgt gacggagttt tggcgtggtt ggtggagggg gggaggaagg      300

agcggtctca tatg                                                        314


<210>  7
<211>  16
<212>  DNA
<213>  Artificial Sequence

<220>
<223>  ZmCPL3v8 (Figure 2)

<400>  7
gtgtgctcta catatg                                                      16


<210>  8
<211>  314
<212>  DNA
<213>  Artificial Sequence

<220>
<223>  ZmCPL3v8 (Figure 2) embedded in SEQ ID NO: 1

<400>  8
gatccgaccc ggtgtgctct acatatgcgt cccgtcaccc ctctcctctt catcctctct      60

tttcttcgcc caaatctcgc gtgctgcctc cgtcctgccc ccagcgcgtg cgctccgatc      120

cgccgggttt cagcgctgcg gctgtcgatt tacgcgattt gtctggcaga atacagttca      180

gagcctcgag cgctgcagat cgaggacctg gtcgtcggat tcgggcgccg cgggtgagac      240

ctaggcgccg gagtttttgt gacggagttt tggcgtggtt ggtggagggg gggaggaagg      300

agcggtctca tatg                                                        314


<210>  9
<211>  15
<212>  DNA
<213>  Artificial Sequence

<220>
<223>  ZmCPL3 TATA-Box (longer version, Figure 3)

<400>  9
ctacatatgc gtccc                                                       15


<210>  10
<211>  56

<212>  DNA
<213>  Artificial Sequence

<220>
<223>  ZmCPL3 promoter (excerpt) (TATA+Y-patch, Figure 4)

<400>  10
ctacatatgc gtcccgtcac ccctctcctc ttcatcctct cttttcttcg cccaaa          56


<210>  11
<211>  9
<212>  DNA
<213>  Artificial Sequence

<220>
<223>  TATA consensus sequence (Figure 5)

<400>  11
ctatawawa                                                              9


<210>  12
<211>  10
<212>  DNA
<213>  Artificial Sequence

<220>
<223>  Y-patch consensus sequence (Figure 6)

<400>  12
cyyyyyyyyc                                                             10


<210>  13
<211>  9
<212>  DNA
<213>  Artificial Sequence

<220>
<223>  TATA box ideal sequence (Figure 7A)

<400>  13
ctataaata                                                              9


<210>  14
<211>  9
<212>  DNA
<213>  Artificial Sequence

<220>
<223>  C1-A mutant (Figure 7A)

<400>  14
atataaata                                                              9


<210>  15
<211>  9
<212>  DNA
<213>  Artificial Sequence

<220>
<223>  C1-T mutant (Figure 7A)

<400>  15
ttataaata                                                                9


<210>  16
<211>  9
<212>  DNA
<213>  Artificial Sequence

<220>
<223>  C1-G mutant (Figure 7A)

<400>  16
gtataaata                                                                9


<210>  17
<211>  9
<212>  DNA
<213>  Artificial Sequence

<220>
<223>  C1-T/T2-C mutant (Figure 7A)

<400>  17
tcataaata                                                                9


<210>  18
<211>  9
<212>  DNA
<213>  Artificial Sequence

<220>
<223>  C1-T/T2-A mutant (Figure 7A)

<400>  18
taataaata                                                                9


<210>  19
<211>  9
<212>  DNA
<213>  Artificial Sequence

<220>
<223>  A6-T mutant (Figure 7A)

<400>  19
ctatatata                                                                9


<210>  20
<211>  9
<212>  DNA
<213>  Artificial Sequence

<220>
<223>  A6-C mutant (Figure 7A)

<400>  20
ctatacata                                                                9

<210>  21
<211>  9
<212>  DNA
<213>  Artificial Sequence

<220>
<223>  A6-G mutant (Figure 7A)

<400>  21
ctatagata                                                                9

<210>  22
<211>  9
<212>  DNA
<213>  Artificial Sequence

<220>
<223>  T8-A mutant (Figure 7A)

<400>  22
ctataaaaa                                                                9

<210>  23
<211>  9
<212>  DNA
<213>  Artificial Sequence

<220>
<223>  T8-C mutant (Figure 7A)

<400>  23
ctataaaca                                                                9

<210>  24
<211>  9
<212>  DNA
<213>  Artificial Sequence

<220>
<223>  T8-G mutant (Figure 7A)

<400>  24
ctataaaga                                                                9

<210>  25
<211>  9
<212>  DNA
<213>  Artificial Sequence

<220>
<223>  A9-G mutant (Figure 7A)

<400>  25
ctataaatg                                                                9

<210>  26
<211>  9
<212>  DNA
<213>  Artificial Sequence

<220>
<223>  A9-C mutant (Figure 7A)

<400>  26
ctataaatc                                                                9


<210>  27
<211>  9
<212>  DNA
<213>  Artificial Sequence

<220>
<223>  A9-T mutant (Figure 7A)

<400>  27
ctataaatt                                                                9


<210>  28
<211>  10
<212>  DNA
<213>  Arabidopsis thaliana

<400>  28
tttcttcttc                                                               10


<210>  29
<211>  10
<212>  DNA
<213>  Artificial Sequence

<220>
<223>  ZmCPL3v9 (Figure 3)

<400>  29
ctacataccc                                                               10


<210>  30
<211>  309
<212>  DNA
<213>  Artificial Sequence

<220>
<223>  ZmCPL3v9 (Figure 3) embedded in SEQ ID NO: 1

<400>  30
gatccgaccc ggcgtgctct acatacccgt cacccctctc ctcttcatcc tctcttttct       60

tcgcccaaat ctcgcgtgct gcctccgtcc tgcccccagc gcgtgcgctc cgatccgccg      120

ggtttcagcg ctgcggctgt cgatttacgc gatttgtctg gcagaataca gttcagagcc      180

tcgagcgctg cagatcgagg acctggtcgt cggattcggg cgccgcgggt gagacctagg      240

cgccggagtt tttgtgacgg agttttggcg tggttggtgg agggggggag gaaggagcgg 300

tctcatatg 309

<210> 31
<211> 307
<212> DNA
<213> Artificial Sequence

<220>
<223> ZmCPL3v10 (Figure 3) embedded in SEQ ID NO: 1

<400> 31
gatccgaccc ggcgtgctcg cgtcccgtca cccctctcct cttcatcctc tcttttcttc 60

gcccaaatct cgcgtgctgc ctccgtcctg cccccagcgc gtgcgctccg atccgccggg 120

tttcagcgct gcggctgtcg atttacgcga tttgtctggc agaatacagt tcagagcctc 180

gagcgctgca gatcgaggac ctggtcgtcg gattcgggcg ccgcgggtga gacctaggcg 240

ccggagtttt tgtgacggag ttttggcgtg gttggtggag ggggggagga aggagcggtc 300

tcatatg 307

<210> 32
<211> 310
<212> DNA
<213> Artificial Sequence

<220>
<223> ZmCPL3v11 (Figure 3) embedded in SEQ ID NO: 1

<400> 32
gatccgaccc ggcgtgctct acgcgtcccg tcacccctct cctcttcatc ctctcttttc 60

ttcgcccaaa tctcgcgtgc tgcctccgtc ctgcccccag cgcgtgcgct ccgatccgcc 120

gggtttcagc gctgcggctg tcgatttacg cgatttgtct ggcagaatac agttcagagc 180

ctcgagcgct gcagatcgag gacctggtcg tcggattcgg gcgccgcggg tgagacctag 240

gcgccggagt ttttgtgacg gagttttggc gtggttggtg gaggggggga ggaaggagcg 300

gtctcatatg 310

<210> 33
<211> 25
<212> DNA
<213> Artificial Sequence

<220>
<223> ZmCPL3v12 (Figure 4)

<400> 33
ctacatatgc gtcccgtcgc ccaaa 25

<210> 34
<211> 283

<212>  DNA
<213>  Artificial Sequence

<220>
<223>  ZmCPL3v12 (Figure 4) embedded in SEQ ID NO: 1

<400>  34
gatccgaccc ggcgtgctct acatatgcgt cccgtcgccc aaatctcgcg tgctgcctcc      60

gtcctgcccc cagcgcgtgc gctccgatcc gccgggtttc agcgctgcgg ctgtcgattt     120

acgcgatttg tctggcagaa tacagttcag agcctcgagc gctgcagatc gaggacctgg     180

tcgtcggatt cgggcgccgc gggtgagacc taggcgccgg agtttttgtg acggagtttt     240

ggcgtggttg gtggaggggg ggaggaagga gcggtctcat atg                       283


<210>  35
<211>  32
<212>  DNA
<213>  Artificial Sequence

<220>
<223>  ZmCPL3v13 (Figure 4)

<400>  35
ctacatatgc gtcccgtcac ccctctcctc tt                                    32


<210>  36
<211>  290
<212>  DNA
<213>  Artificial Sequence

<220>
<223>  ZmCPL3v13 (Figure 4) embedded in SEQ ID NO: 1

<400>  36
gatccgaccc ggcgtgctct acatatgcgt cccgtcaccc ctctcctctt tctcgcgtgc      60

tgcctccgtc ctgcccccag cgcgtgcgct ccgatccgcc gggtttcagc gctgcggctg     120

tcgatttacg cgatttgtct ggcagaatac agttcagagc ctcgagcgct gcagatcgag     180

gacctggtcg tcggattcgg gcgccgcggg tgagacctag gcgccggagt ttttgtgacg     240

gagttttggc gtggttggtg gaggggggga ggaaggagcg gtctcatatg                 290


<210>  37
<211>  31
<212>  DNA
<213>  Artificial Sequence

<220>
<223>  ZmCPL3v12 deletion

<400>  37
cacccctctc ctcttcatcc tctcttttct t                                     31


<210>  38

<211>  24
<212>  DNA
<213>  Artificial Sequence

<220>
<223>  ZmCPL3v13 deletion

<400>  38
catcctctct tttcttcgcc caaa                                              24

## Description

### Technical Field

The present invention relates to novel strategies for gradually decreasing the expression of genes by modifying the promoter sequence. The invention defines modifications in certain promoter elements, which result in a reduced activity of the promoter. Provided are methods for gradually decreasing the expression level of a nucleic acid molecule of interest in a cell, preferably in a plant cell, comprising the step of introducing at least one modification into a promoter sequence, wherein the modification disrupts a core promoter consensus sequence. The modification is either a point mutation or a targeted deletion of one or more defined nucleotide(s). Furthermore, the invention also provides methods for producing a cell or an organism, preferably a plant cell or a plant, having a decreased expression level of a nucleic acid molecule of interest. The invention also relates to a cell and an organism, preferably a plant cell and a plant, obtained by a method according to the invention.

### Background of the invention

The expression levels of many genes in an organism depend on different factors such as developmental stages or physiologic and environmental conditions. The expression of one gene can be induced under certain circumstances and completely shut down if the circumstances change. The starting point for gene expression, the transcription of a gene, is regulated by a range of different mechanisms, which usually involve the promoter region harbouring the transcription start site (TSS). While some promoters are active in all circumstances (constitutive promoters), others are tightly regulated and only respond to certain stimuli. Transcription factors bind to specific DNA sequences and activate or repress transcription (trans-acting factors). Promoter sequences therefore carry a number of binding sites for trans-acting factors, so called cis-regulatory elements, but the functions of some sequence stretches found in promoters are not completely understood yet.

Being able to modulate the expression of certain genes in an organism opens up a range of opportunities to improve biotechnological processes or agricultural yields. Therefore, new technologies are continuously sought, which allow to specifically fine-tune the expression levels of a target gene. Promoters are an obvious target for such approaches, but up to date, there is still little known about the possibilities of activating endogenous promoters or gradually decreasing their activity by minimal modification.

It is known that increased expression can be achieved by using strong promoters, e.g. the 35S promoter. Different translation enhancing elements have been described to be useful for expressing high levels of protein in plant cells, as parts of transgenes, or in viral expression vectors (e.g. the 5' untranslated leader of tobacco mosaic virus RNA which consists of a 68-base sequence (see Ofoghi et al., 2005. Comparison of tobacco etch virus and tobacco mosaic virus enhancers for expression of human calcitonin gene in transgenic potato plant. In *Key Engineering Materials* (Vol. 277, pp. 7-11). Trans Tech Publications.). However, these elements are relatively large. The same is true for enhancing promoter elements like the 35S enhancer, or introns reported to increase expression, e.g. adh1 intron from Zea mays (Callis et al, 1987. Introns increase gene expression in cultured maize cells. Genes & development, 1(10), 1183-1200.).

Crop traits can be improved by increased ectopic expression of a trait gene. As an example, Sun et al. (Nature comm., 2017, doi: 10.1038/ncomms14752) reported that increased expression of maize PLASTOCHRON1 enhances biomass and seed yield. They increased expression by a transgenic approach, using the GA2ox promoter. This transgenic approach to increase ectopic expression of a trait gene has the limitation that planting of transgenic plants has high regulatory requirements.

Recently, Zhang et al. found in the genus Malus an allelic variation of the Iron-Regulated Transporter1 (IRT1) promoter in which a TATA box insertion has been identified (Plant Physiology, 2017, Vol. 173, 715-727, doi: 10.1104/pp.16.01504). Further results suggest that this insertion seems to be causative for a slight upregulation of the promoter activity (~1.5 fold). It is also possible that the increased promoter activity is caused by the increased expression of the potential TATA-box binding proteins TFIID, which activates also the IRT1 promoter.

In some instances, it may be desirable to reduce the expression of a gene but a total knockout may be detrimental or even lethal for the organism under investigation. For example, the expression of a pleiotropic gene plays a role in the manifestation of several unrelated traits so that its knockout may have undesired side effects. Gene knockdown techniques, which reduce or transiently reduce the expression of a gene largely rely on RNA interference techniques affecting gene expression by silencing. Such techniques usually decrease the expression of a target gene to a large extent but do not completely shut it down. However, it is currently not feasible to adjust the expression of a target gene to a desired level using gene silencing. Moreover, a stable expression of gene silencing constructs in a cell is usually achieved by the introduction of transgenes, which is subject to strong regulations in certain countries and therefore often not applicable for commercial purposes.

It was an object of the present invention to provide techniques for gradually decreasing the expression level of a target gene of interest.

In particular, it was an object of the present invention to provide means and methods to fine-tune the expression of a target gene of interest and preferably to be able to adjust it to a desired level.

It was also an object of the present invention that the method should involve minimal genetic modification of the target cell and in particular not require the introduction of transgenes in order to avoid regulatory limitations for its use.

### Summary of the invention

In one aspect, the present invention relates to a method for gradually decreasing the expression level of a nucleic acid molecule of interest in at least one cell, preferably a plant cell, comprising the following steps:
(i) introducing at least one modification into a promoter sequence of the nucleic acid molecule of interest, wherein the modification is at least one, and preferably exactly one, point mutation and/or a targeted deletion of one or more defined and consecutive nucleotides, wherein the modification disrupts at least one core promoter consensus sequence; and
(ii) obtaining at least one cell showing a decreased expression level of a nucleic acid molecule of interest compared to the expression level of the nucleic acid molecule of interest under the control of an unmodified control promoter.

In one embodiment of the various aspects of the present invention, in the method described above, the core promoter consensus sequence is a TATA box motif, a Y-patch motif, an initiator element, or a downstream promoter element and/or the at least one modification is a point mutation disrupting a TATA box motif and/or the at least one modification is a targeted deletion of one or more consecutive nucleotides comprising one or more nucleotides forming or being part of a TATA box motif in the core promoter sequence.

In another embodiment of the various aspects of the present invention, in the method according to any of the embodiments described above, the at least one modification is a targeted deletion of one or more nucleotides comprising at least one, two, three, four, five, six, seven, eight or nine consecutive nucleotides forming or being part of a TATA box motif in the core promoter sequence and/or, the at least one modification is a targeted deletion of one or more nucleotides comprising at least one, two, three, four, five, six, seven, eight, nine, ten, eleven, twelve, thirteen, fourteen, fifteen, twenty, twenty-five, thirty, thirty-five, forty, forty-five, or at least fifty consecutive nucleotides of a Y-patch in the core promoter sequence.

In a further embodiment of the various aspects of the present invention, in the method according to any of the embodiments described above, the expression level of the nucleic acid molecule of interest is decreased by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10% compared to the expression level of the nucleic acid molecule of interest under the control of an unmodified control promoter.

In one embodiment of the various aspects of the present invention, in the method according to any of the embodiments described above,
(a) the expression level of the nucleic acid molecule of interest is decreased by 40 to 80%, preferably 40 to 60% , compared to the expression level of the nucleic acid molecule of interest under the control of an unmodified control promoter when the at least one modification is a point mutation affecting the one or the two consecutive nucleotides located at the 5'-end and/or at the 3'-end of a TATA box motif or wherein the modification is a targeted deletion of consecutive nucleotides comprising 20 to 60%, preferably 30 to 50% of the nucleotides forming a Y-patch motif; or
(b) the expression level of the nucleic acid molecule of interest is decreased by 50 to 95%, preferably 70 to 95% compared to the expression level of the nucleic acid molecule of interest under the control of an unmodified control promoter when the at least one modification is a point mutation affecting one or two consecutive nucleotides located at least one nucleotide apart from the 5'-end and/or the 3'-end of a TATA box motif, or wherein the modification is a targeted deletion of two or more nucleotides comprising at least two, at least three, at least four, at least five, at least six or at least seven nucleotides forming or being part of a TATA box motif, or wherein the modification is a targeted deletion of consecutive nucleotides comprising 75 to 95%, preferably 80 to 90% of the nucleotides forming a Y-patch motif.

In another embodiment of the various aspects of the present invention, in the method according to any of the embodiments described above, the at least one modification is introduced by mutagenesis or by site-specific modification techniques using a site-specific nuclease or an active fragment thereof and/or a base editor.

In a further embodiment of the various aspects of the present invention, in the method according to any of the embodiments described above, step (i) includes: introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

In another aspect, the present invention relates to a method for producing a cell or an organism, preferably a plant cell or a plant, having a decreased expression level of a nucleic acid molecule of interest, comprising the steps:
(i) providing a cell comprising the nucleic acid molecule of interest, which is expressed under the control of a promoter;
(ii) introducing at least one modification into the promoter sequence of the nucleic acid molecule of interest, wherein the modification is at least one, and preferably exactly one point mutation and/or a targeted deletion of one or more defined and consecutive nucleotides, wherein the modification disrupts at least one core promoter consensus sequence, and
(iii) obtaining a cell having a decreased expression level of the nucleic acid molecule of interest compared to the expression level of the nucleic acid molecule of interest under the control of an unmodified control promoter and optionally, culturing the cell to obtain an organism having a decreased expression level of a nucleic acid molecule of interest.

In one embodiment of the method for producing a cell or an organism described above, the core promoter consensus sequence is a TATA box motif, a Y-patch motif, an initiator element, or a downstream promoter element and/or the at least one modification is a point mutation disrupting a TATA box motif and/or the at least one modification is a targeted deletion of one or more consecutive nucleotides comprising one or more nucleotides forming or being part of a TATA box motif in the core promoter sequence.

In another embodiment of the method for producing a cell or an organism according to any of the embodiments described above, the at least one modification is a targeted deletion of one or more nucleotides comprising at least one, two, three, four, five, six, seven, eight or nine consecutive nucleotides forming or being part of a TATA box motif in the core promoter sequence and/or, the at least one modification is a targeted deletion of one or more nucleotides comprising at least one, two, three, four, five, six, seven, eight, nine, ten, eleven, twelve, thirteen, fourteen, fifteen, twenty, twenty-five, thirty, thirty-five, forty, forty-five, or at least fifty consecutive nucleotides of a Y-patch in the core promoter sequence.

In a further embodiment of the method for producing a cell or an organism according to any of the embodiments described above, the expression level of the nucleic acid molecule of interest is decreased by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10% compared to the expression level of the nucleic acid molecule of interest under the control of an unmodified control promoter.

In one embodiment of the method for producing a cell or an organism according to any of the embodiments described above,
(a) the expression level of the nucleic acid molecule of interest is decreased by 40 to 80%, preferably 40 to 60%, compared to the expression level of the nucleic acid molecule of interest under the control of an unmodified control promoter when the at least one modification is a point mutation affecting the one or the two consecutive nucleotides located at the 5'-end and/or at the 3'-end of a TATA box motif or wherein the modification is a targeted deletion of consecutive nucleotides comprising 20 to 60%, preferably 30 to 50% of the nucleotides forming a Y-patch motif; or
(b) the expression level of the nucleic acid molecule of interest is decreased by 50 to 95%, preferably 75 to 95%, compared to the expression level of the nucleic acid molecule of interest under the control of an unmodified control promoter when the at least one modification is a point mutation affecting one or two consecutive nucleotides located at least one nucleotide apart from the 5'-end and/or the 3'-end of a TATA box motif, or wherein the modification is a targeted deletion of two or more nucleotides comprising at least two, at least three, at least four, at least five, at least six or at least seven nucleotides forming or being part of a TATA box motif, or wherein the modification is a targeted deletion of consecutive nucleotides comprising 75 to 95%, preferably 80 to 90% of the nucleotides forming a Y-patch motif.

In another embodiment of the method for producing a cell or an organism according to any of the embodiments described above, the at least one modification is introduced in step (ii) by mutagenesis or by site-specific modification techniques using a site-specific nuclease or an active fragment thereof and/or a base editor, preferably wherein step (ii) includes: introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

In another aspect, the present invention relates to a cell obtained or obtainable by a method for producing a cell or an organism according to any of the embodiments described above. In yet another aspect, the present invention relates to an organism or a part thereof, preferably a plant or part of a plant obtained or obtainable by culturing a cell according to the aspect described above.

### Brief description of the Drawings

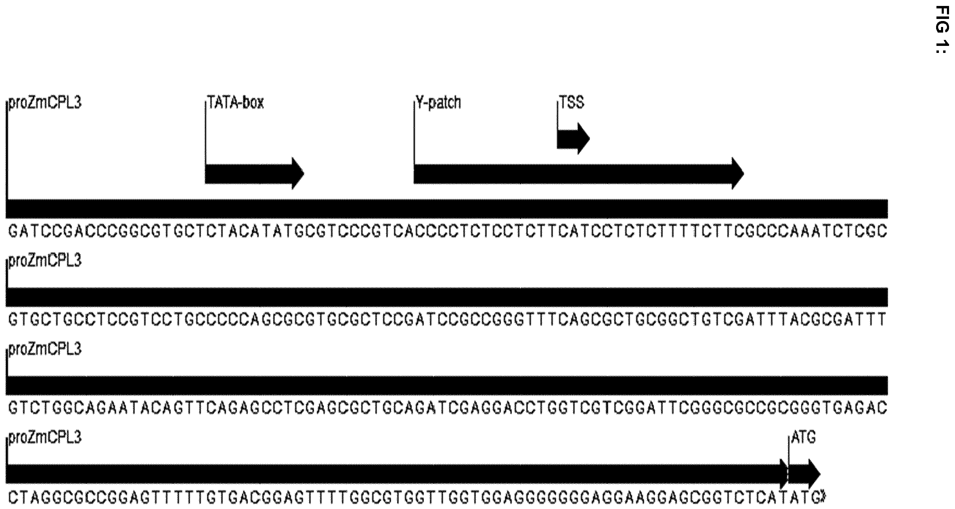

**Figure 1** shows the structure of the 3'-part of the ZmCPL3 promoter. The sequence is given in SEQ ID NO: 1. The transcription start site (TSS), the TATA-box, the Y-patch and the translation start site (ATG) are indicated.

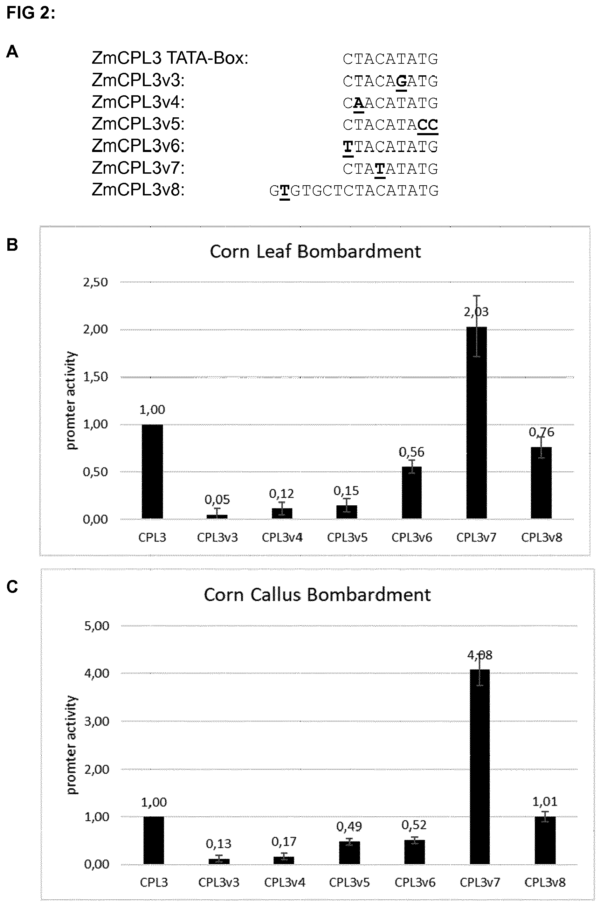

**Figure 2** shows repression of the ZmCPL3 promoter as consequence of distinct point mutations in the TATA-box. The sequence of the TATA box motif of the ZmCPL3 promoter is located from position 19 to 27 of SEQ ID NO: 1, the sequence of the ZmCPL3v3 mutant in the 3'-part of the promoter sequence of SEQ ID NO: 1 is given in SEQ ID NO: 2, the sequence of the ZmCPL3v4 mutant in the 3'-part of the promoter sequence of SEQ ID NO: 1 is given in SEQ ID NO: 3, the sequence of the ZmCPL3v5 mutant in the 3'-part of the promoter sequence of SEQ ID NO: 1 is given in SEQ ID NO: 4, the sequence of the ZmCPL3v6 mutant in the 3'-part of the promoter sequence of SEQ ID NO: 1 is given in SEQ ID NO: 5, the sequence of the ZmCPL3v7 mutant in the 3'-part of the promoter sequence of SEQ ID NO: 1 is given in SEQ ID NO: 6 and the sequence of the ZmCPL3v8 mutant (SEQ ID NO: 7) in the 3'-part of the promoter sequence of SEQ ID NO: 1 is given in SEQ ID NO: 8. (A) displays the point mutations introduced into the different variants of the ZmCPL3 promoter in bold and underlined. (B) shows measurement of promoter activity by applying corn leaf bombardment as transient test system and (C) shows measurement of promoter activity by applying corn callus bombardment as transient test system. The promoter activity is quantified with respect to the activity of the unmodified promoter ZmCPL3, which therefore has an activity of 1.

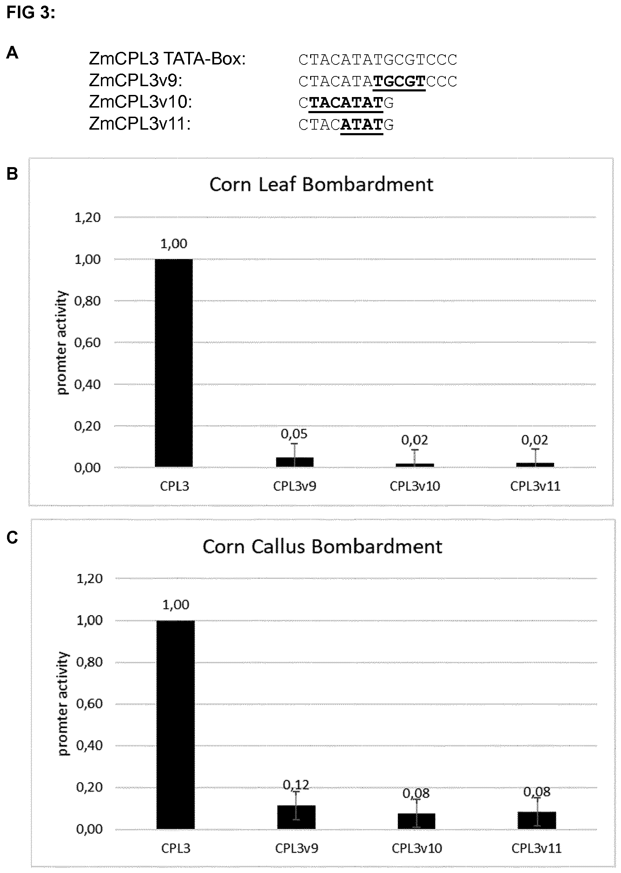

**Figure 3** shows repression of the ZmCPL3 promoter as consequence of small deletions in the TATA-box. The sequence spanning the ZmCPL3 TATA box motif and a 3'-flanking region is given in SEQ ID NO: 9 (corresponds to the nucleotide positions 19 to 33 of SEQ ID NO: 1), the ZmCPL3v9 mutant (SEQ ID NO: 29) corresponds to SEQ ID NO: 9, wherein the bold and underlined nucleotides have been deleted (SEQ ID NO: 30 shows ZmCPL3v9 mutant in the 3'-part of the promoter sequence of SEQ ID NO: 1), the ZmCPL3v10 mutant given in the 3'-part of the promoter sequence of SEQ ID NO: 1 (SEQ ID NO: 31) and the ZmCPL3v11 mutant given in the 3'-part of the promoter sequence of SEQ ID NO: 1 (SEQ ID NO: 32), both correspond to the TATA box motif of the ZmCPL3 promoter as shown in Fig. 2, wherein the respective bold and underlined nucleotides have been deleted. (A) displays the small deletions done in the different variants of the ZmCPL3 promoter in bold and underlined. (B) shows measurement of promoter activity by applying corn leaf bombardment as transient test system and (C) shows measurement of promoter activity by applying corn callus bombardment as transient test system. The promoter activity is quantified with respect to the activity of the unmodified promoter ZmCPL3, which therefore has an activity of 1.

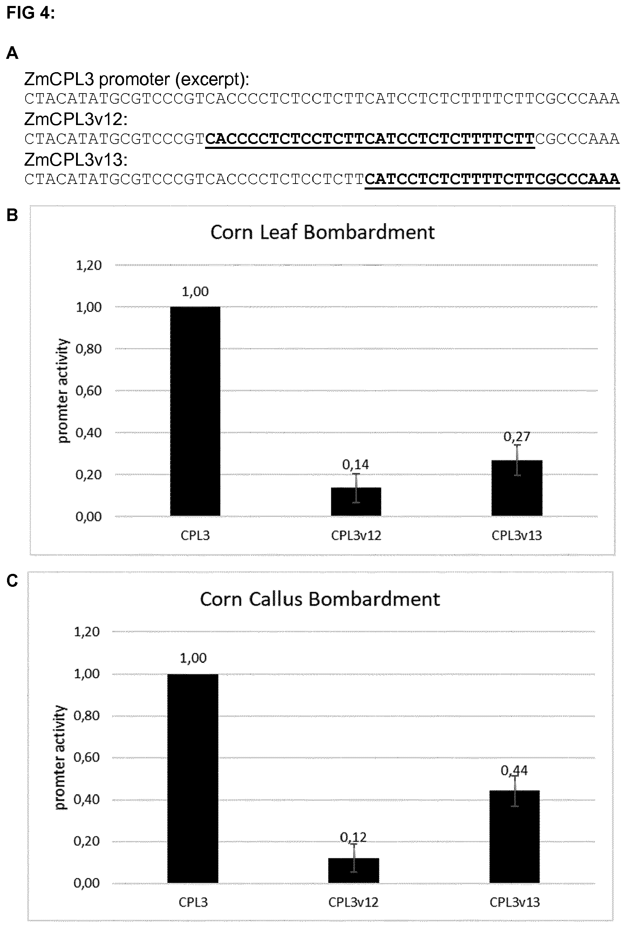

**Figure 4** shows repression of the ZmCPL3 promoter as consequence of deletions in the Y-patch. The ZmCPL3 promoter region including the TATA box and the Y-patch is given in SEQ ID NO: 10 (corresponds to the nucleotide position 19-74 of SEQ ID NO: 1), the ZmCPL3v12 mutant (SEQ ID NO: 33) given in the 3'-part of the promoter sequence of SEQ ID NO: 1 (SEQ ID NO: 34) and the ZmCPL3v13 mutant (SEQ ID NO: 35) given in the 3'-part of the promoter sequence of SEQ ID NO: 1 (SEQ ID NO: 36), both correspond to SEQ ID NO: 10, wherein the respective bold and underlined nucleotides have been deleted. (A) displays the deletions (SEQ ID NOs: 37 and 38) done in the different variants of the ZmCPL3 promoter in bold and underlined. (B) shows measurement of promoter activity by applying corn leaf bombardment as transient test system and (C) shows measurement of promoter activity by applying corn callus bombardment as transient test system. The promoter activity is quantified with respect to the activity of the unmodified promoter ZmCPL3, which therefore has an activity of 1.

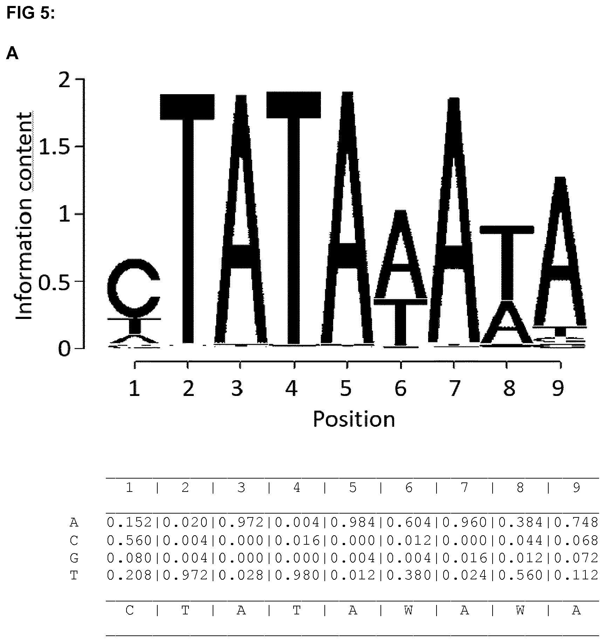

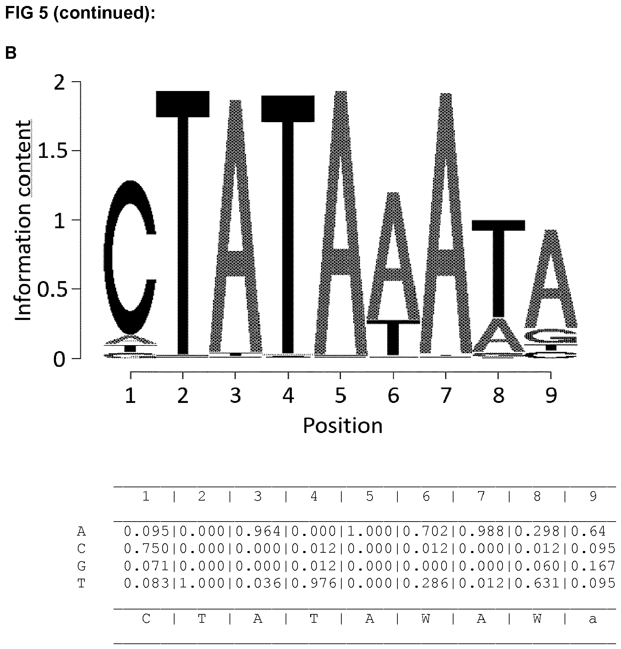

**Figure 5** shows nucleotide frequencies matrices and motif logos for TATA box consensus of dicotyledonous plants (**A**) and monocotyledonous plants (**B**) (Shahmuradov et al. (2003). The consensus sequence for the TATA box motif is given in SEQ ID NO: 11, wherein W stands for A or T. PlantProm: a database of plant promoter sequences. Nucleic acids research, 31(1), 114-117.; http://linux1.softberry.com/berry.phtml/freedownloadhelp/viewers/gmv/berry.phtml?topic=plantprom&group=data&subgroup=plantprom).

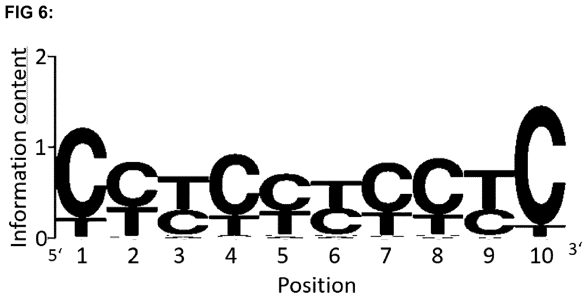

**Figure 6** shows the motif logo of a Y-patch (pyrimidine patch) in plant promoters (Kumari S, Ware D (2013) Genome-Wide Computational Prediction and Analysis of Core Promoter Elements across Plant Monocots and Dicots. PLoS ONE 8(10): e79011. doi:10.1371/journal.pone.0079011, Civan P, Svec M (2009) Genome-wide analysis of rice (Oryza sativa L. subsp. japonica) TATA box and Y Patch promoter elements. Genome/National Research Council Canada = Genome/Conseil national de recherches Canada 52: 294-297) The consensus sequence for the Y-patch is given in SEQ ID NO: 12, wherein Y stands for C or T.

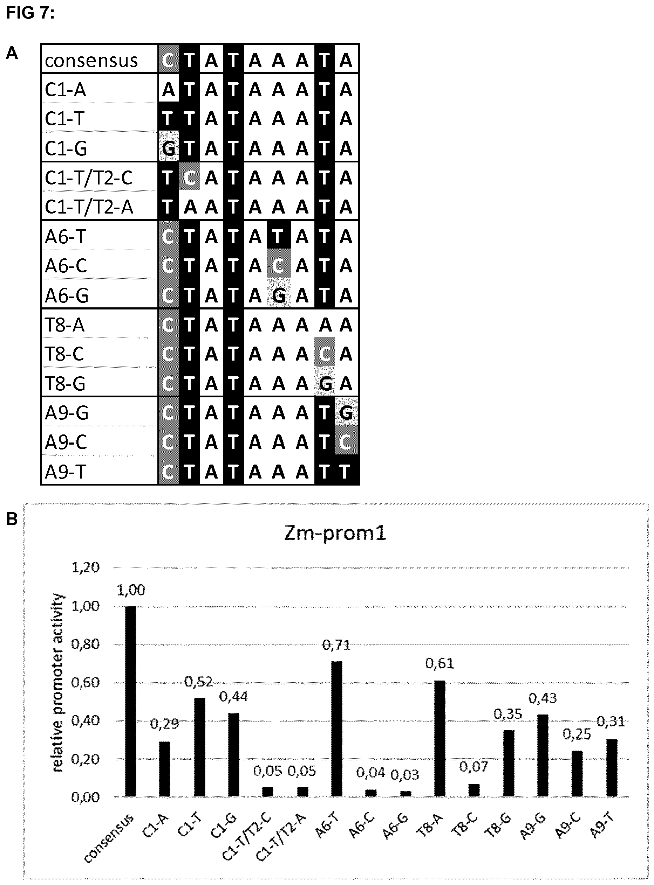

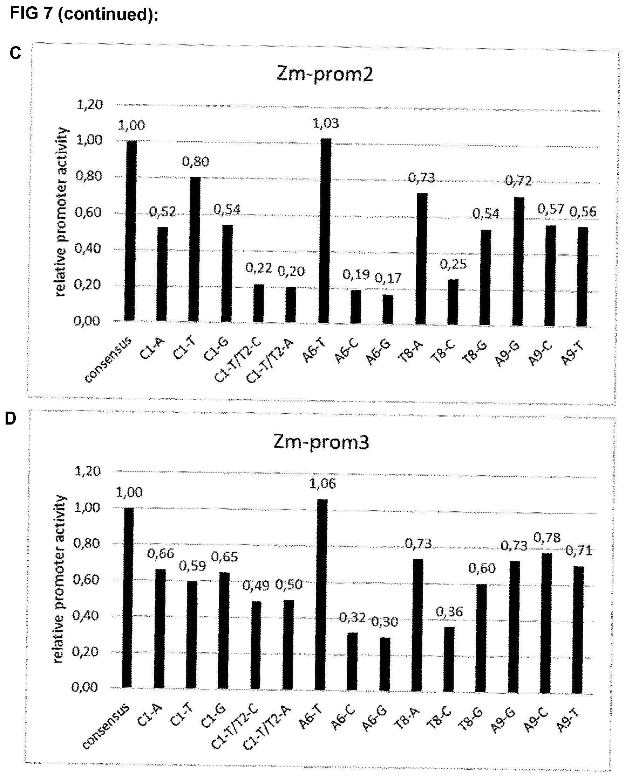

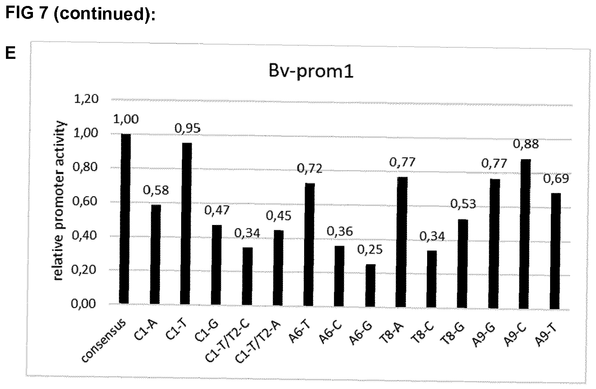

**Figure 7** shows the repressive effect of mutations in a perfect consensus TATA-box motif (**A**) on promoter activity. The sequence of the perfect TATA-box motif is given in SEQ ID NO: 13, while SEQ ID NOs: 14 to 27 represent the mutants C1-A to A9-T. (**B-D**) shows measurement of promoter activity of tree different corn promoters by applying corn leaf bombardment as transient test system, while (**E**) shows measurement of promoter activity of a sugar beet promoter by applying sugar beet leaf bombardment as transient test system.

### Definitions

The term "gene expression" or "expression" as used herein refers to the conversion of the information, contained in a gene or nucleic acid molecule, into a "gene product" or "expression product". A "gene product" or "expression product" can be the direct transcriptional product of a gene or nucleic acid molecule (e.g., mRNA, tRNA, rRNA, antisense RNA, ribozyme, structural RNA or any other type of RNA) or a protein produced by translation of an mRNA. Gene products or expression products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

A "decreased expression level of a nucleic acid molecule of interest" is observed, when the expression of the nucleic acid molecule of interest is lower when the promoter controlling the expression has been modified according to the present invention than when the nucleic molecule of interest is expressed under the same conditions under the control of the unmodified promoter. The expression level under the control of the unmodified promoter is therefore considered to be 100% so that the decrease can be given as a percentage. As a result of the modification, the expression can be decreased "gradually", which means that it can be adjusted to a level, i.e. by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10%. A "decreased expression" or a "decreased expression level", however, is not a complete shutdown of gene expression, i.e. a decrease by 100%, but the gene is still expressed to at least 1%, at least 2%, at least 3%, at least 4%, at least 5%, at least 6%, at least 7%, at least 8%, at least 9% or at least 10% of the expression under the control of the unmodified promoter.

A "promoter" or a "promoter sequence" refers to a DNA sequence capable of controlling and/or regulating expression of a coding sequence, i.e., a gene or part thereof, or of a functional RNA, i.e. a RNA which is active without being translated, for example, a miRNA, a siRNA, an inverted repeat RNA or a hairpin forming RNA. A promoter is usually located at the 5' part of a gene. Promoters can have a broad spectrum of activity, but they can also have tissue or developmental stage specific activity. For example, they can be active in cells of roots, seeds and meristematic cells, etc. A promoter can be active in a constitutive way, or it can be inducible. The induction can be stimulated by a variety of environmental conditions and stimuli. Often promoters are highly regulated. A promoter of the present disclosure may include an endogenous promoter natively present in a cell, or an artificial or transgenic promoter, either from another species, or an artificial or chimeric promoter, i.e. a promoter that does not naturally occur in nature in this composition and is composed of different promoter elements. The process of transcription begins with the RNA polymerase (RNAP) binding to DNA in the promoter region, which is in the immediate vicinity of the transcription start site (TSS) at the position +1. From analysis in Arabidopsis thaliana the most frequently observed sequence at this position is CA, and TA was the second. There is a strong preference of a dimer sequence at the -1/+1 position. It has been clearly shown that most of the TSS is A or G, and the -1 position is likely to be C or T. This YR Rule (Y: C or T, R: A or G) applies to as many as 77% of the Arabidopsis promoters that is a much higher frequency than expected random appearance (25%) (Yamamoto et al. (2007). Identification of plant promoter constituents by analysis of local distribution of short sequences. BMC genomics, 8(1), 67.). A typical promoter sequence is thought to comprise some regulatory sequence motifs positioned at specific sites relative to the TSS. These cis-regual-tory elements are e.g. binding sites for transacting factors such as transcription factors. The structure of eukaryotic promoters may be rather complex as they have several different sequence motifs, such as TATA box, INR box, BRE, CCAAT-box and GC-box (Bucher P., J. Mol. Biol. 1990 Apr 20; 212(4):563-78.) or Y Patch promoter elements (Yamamoto et al. (2007). Identification of plant promoter constituents by analysis of local distribution of short sequences. BMC genomics, 8(1), 67; Civáň, P., & Švec, M. (2009). Genome-wide analysis of rice (Oryza sativa L. subsp. japonica) TATA box and Y Patch promoter elements. Genome, 52(3), 294-297.). Promoters can be of varying length and may span more than a thousand nucleotides. Finally, promoter architectures and function differ in different taxa. In particular, there are huge differences between eukaryotic and prokaryotic promoters, but also eukaryotic promoters, e.g., plant promoters and mammalian cell promoters, differ in structure, function and the regulatory network within the cell.

A "core promoter" or "core promoter sequence" refers to a part of a promoter, which comprises several important promoter elements, such as the transcription start site (TSS), TATA box, Y-patch, initiator element and downstream promoter element. A "core promoter consensus sequence" refers to a sequence of a promoter element, which is defined by one or more conserved motifs that can be used to identify the promoter element.

A "modification" of a (nucleic acid) sequence in the context of the present invention refers to any change of a (nucleic acid) sequence that results in at least one difference in the (nucleic acid) sequence distinguishing it from the original sequence. In particular, a modification can be achieved by insertion or addition of one or more nucleotide(s), or substitution or deletion of one or more nucleotide(s) of the original sequence or any combination of these.

A "targeted deletion" refers to a deletion of predetermined nucleotides in a sequence. The introduction of a targeted deletion therefore requires the use of site-specific techniques, which allow a precise removal of the target nucleotides, while leaving the flanking nucleotides unaffected. The removed nucleotides are "defined" and "consecutive" meaning that a 3'-end and a 5'-end for a sequence of adjacent nucleotides to be removed has been precisely predetermined.

A modification "disrupts" a core promoter consensus sequence, when it changes the sequence so that it differs more from a defined consensus sequence. In particular, a modification disrupts a TATA box motif, when the sequence is altered with respect to a TATA box consensus sequence as defined below. On the other hand, a mutation in a TATA box motif, which alters the sequence so that it is more similar to the respective consensus sequence, is not a disruption but an enhancement, which will result in a higher expression level. It is noted in this context, that a core promoter consensus sequence before modification according to the invention does not necessarily need to correspond exactly to the consensus sequence but it may be already differ in one or more positions. After a modification, which disrupts the core promoter consensus sequence, however, the sequence is even less similar to the consensus sequence. Preferably, a disruption does not result in the complete removal (deletion) or inactivation of the promoter element defined by the consensus sequence but it results in a reduced expression level of the nucleic acid molecule under the control of the modified promoter compared to the unmodified promoter.

An "unmodified control promoter" is a promoter, which has not been modified according to the invention. In the method of gradually decreasing the expression level of a nucleic acid molecule of interest and the method for producing a cell or an organism having a decreased expression level of a nucleic acid molecule of interest according to the invention, the original promoter present before the introduction of the modification may be used as control promoter. In order to determine a decrease of expression level, the nucleic acid molecule of interest is expressed under the same conditions (environmental conditions, developmental stage etc.) under the control of the control promoter and under the control of the modified promoter. The decrease in expression level can then be expressed as a percentage, wherein the expression level of the nucleic acid molecule of interest under the control of the unmodified control promoter corresponds to 100%.

A "TATA box motif" refers to a sequence found in many core promoter regions of eukaryotes. The TATA box motif is usually found within 100 nucleotides upstream of the transcription start site. In plant promoters, the TATA-box motif is found about 25 to 40 nt, preferably 31 to 32 nt, upstream of the transcription start site. The TATA box motif also represents the binding site for TBP (TATA box binding protein). "TATA box consensus sequences" for dicotyledonous plants and monocotyledonous plants are defined in Figure 5 as nucleotide frequencies matrices and motif logos. The consensus sequence is given in SEQ ID NO: 11. An ideal TATA box motif is given in SEQ ID NO: 13.

The nucleotides located at the 5'- or the 3'-end of the TATA box motif correspond to positions 1 or 9, respectively, of the consensus sequence of SEQ ID NO: 11 and the nucleotides located at least one nucleotide apart from the 5'- or the 3'-end of the TATA box motif correspond to positions 2 to 8 of the consensus sequence of SEQ ID NO: 11.

A "Y-patch promoter element" or "pyrimidine patch promoter element" or "Y-patch" or "pyrimidine patch" refers to a sequence found in many promoters of higher plants. A typical Y-patch is composed of C and T (pyrimidine) (Yamamoto et al. (2007). Identification of plant promoter constituents by analysis of local distribution of short sequences. BMC genomics, 8(1), 67.). AY-patch can be detected by LDSS (local distribution of short sequences) analysis as well as by a search for consensus sequence from plant promotors, preferably core promoters, by MEME and AlignACE (examplary motif for A. thaliana: TTTCTTCTTC (SEQ ID NO: 28) (Molina & Grotewold. Genome wide analysis of Arabidopsis core promoters. BMC Genomics. 2005;6:25.). The Y-patch is usually found within 100 nucleotides upstream of the transcription start site (postion -1 to -100 relative to TSS), preferably at a position between -10 to -60 relative to TSS.

An "initiator element (Inr)" is a core promoter sequence, which has a similar function as the TATA box and can also enable transcription initiation in the absence of a TATA box. It facilitates the binding of transcription factor II D, which is part of the RNA polymerase II preinitiation complex. The Inr encompasses the TSS and may contain a dimer motif (C/T A/G).

A "downstream promoter element (DPE)" is a core promoter sequence, which also plays a role in transcription initiation and is located downstream of the TSS. It is recognized by the transcription factor II D together with the Inr.

The "transcription start site" refers to the first nucleotide of a DNA sequence that is transcribed. This nucleotide is assigned the position +1 within the promoter. "Upstream" and "downstream" relate to the 5' to 3' direction in which RNA transcription takes place. Upstream is toward the 5' end of the RNA molecule and downstream is toward the 3' end. On the DNA from which transcription takes place, upstream is toward the 5' end of the coding strand for the gene in question and downstream is toward the 3' end.

The term "one or more" includes "one or two", "one, two or three", "one, two, three or four" and "one, two, three, four or five", but has generally the meaning of "at least one".

The terms "plant" or "plant cell" as used herein refer to a plant organism, a plant organ, differentiated and undifferentiated plant tissues, plant cells, seeds, and derivatives and progeny thereof. Plant cells include without limitation, for example, cells from seeds, from mature and immature cells or organs, including embryos, meristematic tissues, seedlings, callus tissues in different differentiation states, leaves, flowers, roots, shoots, male or female gametophytes, sporophytes, pollen, pollen tubes and microspores, protoplasts, macroalgae and microalgae. The cells can have any degree of ploidity, i.e. they may either be haploid, diploid, tetraploid, hexaploid or polyploid.

The term "mutagenesis" refers to a method by which random mutations are introduced into genomic material into one or more cell(s) or organism(s) or into a population of organisms. This can be achieved by exposing the cell(s) or organism(s) to mutagens, such as UV radiation or mutagenic chemicals. Mutagenic chemicals include alkylating agents such as *N*-ethyl-*N*-nitrosourea. Ethyl methanesulfonate (EMS) is also commonly used to generate mutants. Subsequently, the cells or organisms can be screened and selected for desired mutations. A well-established method for mutagenesis is TILLING (targeting induced local lesions in genomes), which combines a standard mutagenesis e.g. using EMS, with a sensitive DNA screening-technique to identify point mutations.

A "site-specific modification technique" is any established technique to introduce a targeted modification at a predetermined location. Such techniques utilize any tools for site-specific modification, i.e. insertion, addition, substitution or deletion, of nucleic acid sequences known to the skilled person. Examples are, in particular, "site-specific effectors" such as nucleases, nickases, recombinases, transposases, base editors or molecular complexes including these tools. These effectors have the capacity to introduce a single- or double-strand cleavage into a genomic target site, or have the capacity to introduce a targeted modification, including a point mutation, an insertion, or a deletion, into a genomic target site of interest. A site-specific effector can act on its own, or in combination with other molecules as part of a molecular complex. The site-specific effector can be present as fusion molecule, or as individual molecules associating by or being associated by at least one of a covalent or non-covalent interaction so that the components of the site-specific effector complex are brought into close physical proximity. The complex may include a repair template to make a targeted sequence conversion or replacement at the target site.

A "repair template (RT)" represents a single-stranded or double-stranded nucleic acid sequence, which can be provided during any genome editing causing a double-strand or single-strand DNA break to assist the targeted repair of said DNA break by providing a RT as template of known sequence assisting homology-directed repair.

A "site-specific nuclease" refers to a nuclease or an active fragment thereof, which is capable to specifically recognize and cleave DNA at a certain location. This location is herein also referred to as a "predetermined location". Such nucleases typically produce a double strand break (DSB), which is then repaired by nonhomologous end-joining (NHEJ) or homologous recombination (HR). The nucleases include zinc-finger nucleases, transcription activator-like effector nucleases, CRISPR/Cas systems, including CRISPR/Cas9 systems, CRISPR/Cpf1 systems, CRISPR/C2C2 systems, CRISPR/CasX systems, CRISPR/CasY systems, CRISPR/Cmr systems, CRISPR/MAD7 systems, CRISPR/CasZ systems, engineered homing endonucleases, recombinases, transposases and meganucleases, and/or any combination, variant, or catalytically active fragment thereof.

A "CRISPR nuclease", as used herein, is any nuclease which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. Said DNA recognition can be PAM (protospacer adjacent motif) dependent. CRISPR nucleases having optimized and engineered PAM recognition patterns can be used and created for a specific application. The expansion of the PAM recognition code can be suitable to target site-specific effector complexes to a target site of interest, independent of the original PAM specificity of the wild-type CRISPR-based nuclease. Cpf1 variants can comprise at least one of a S542R, K548V, N552R, or K607R mutation, preferably mutation S542R/K607R or S542R/K548V/N552R in AsCpf1 from *Acidaminococcus.* Furthermore, modified Cas or Cpf1 variants or any other modified CRISPR effector variants, e.g., Cas9 variants, can be used according to the methods of the present invention as part of a base editing complex, e.g. BE3, VQR-BE3, EQR-BE3, VRER-BE3, SaBE3, SaKKH-BE3 (see Kim et al., Nat. Biotech., 2017, doi:10.1038/nbt.3803). Therefore, according to the present invention, artificially modified CRISPR nucleases are envisaged, which might indeed not be any "nucleases" in the sense of double-strand cleaving enzymes, but which are nickases or nuclease-dead variants, which still have inherent DNA recognition and thus binding ability. Suitable Cpf1-based effectors for use in the methods of the present invention are derived from *Lachnospiraceae bacterium* (LbCpf1, e.g., NCBI Reference Sequence: WP_051666128.1), or from *Francisella tularensis* (FnCpf1, e.g., UniProtKB/Swiss-Prot: A0Q7Q2.1). Variants of Cpf1 are known (cf. Gao et al., BioRxiv, dx.doi.org/10.1101/091611). Variants of AsCpf1 with the mutations S542R/K607R and S542R/K548V/N552R that can cleave target sites with TYCV/CCCC and TATV PAMs, respectively, with enhanced activities *in vitro* and *in vivo* are thus envisaged as site-specific effectors according to the present invention. Genome-wide assessment of off-target activity indicated that these variants retain a high level of DNA targeting specificity, which can be further improved by introducing mutations in non-PAM-interacting domains. Together, these variants increase the targeting range of AsCpf1 to one cleavage site for every -8.7 bp in non-repetitive regions of the human genome, providing a useful addition to the CRISPR/Cas genome engineering toolbox (see Gao et al., supra).

A "base editor" as used herein refers to a protein or a fragment thereof having the same catalytic activity as the protein it is derived from, which protein or fragment thereof, alone or when provided as molecular complex, referred to as base editing complex herein, has the capacity to mediate a targeted base modification, i.e., the conversion of a base of interest resulting in a point mutation of interest. Preferably, the at least one base editor in the context of the present invention is temporarily or permanently linked to at least one site-specific effector, or optionally to a component of at least one site-specific effector complex. The linkage can be covalent and/or non-covalent.

### Detailed Description

The present invention relates to several aspects to establish a new technology to gradually decrease the expression level of a nucleic acid molecule of interest. By introducing certain modifications in the core promoter sequence, it becomes possible to reduce the expression of the nucleic acid of interest by a desired amount.

In a first aspect, a method for gradually decreasing the expression level of a nucleic acid molecule of interest in at least one cell, preferably a plant cell, is provided, comprising the following steps:
(i) introducing at least one modification into a promoter sequence of the nucleic acid molecule of interest, wherein the modification is at least one, and preferably exactly one, point mutation and/or a targeted deletion of one or more defined and consecutive nucleotides, wherein the modification disrupts at least one core promoter consensus sequence; and
(ii) obtaining at least one cell showing a decreased expression level of a nucleic acid molecule of interest compared to the expression level of the nucleic acid molecule of interest under the control of an unmodified control promoter.

A promoter core sequence comprises several core promoter elements. These core promoter elements play an essential role in transcription initiation as the first step of gene expression. Core promoter elements can be identified by certain conserved motifs, which define a core promoter consensus sequence. It has been discovered now that the actual sequence of the respective motifs in a given promoter is characteristic for the activity of the promoter and thus for the expression level of the expression product under its control. Modification of the sequence of the motifs therefore impacts the expression levels.

The promoter controlling the expression of the nucleic acid molecule of interest before the modification is introduced in step i) may contain a motif, which corresponds to the consensus sequence or it may contain a sequence which differs in one or more positions from the consensus sequence. For an increase in promoter activity, resulting in higher expression levels of the target gene, the sequence of the motif can be altered in a way that it becomes more similar to the consensus sequence of the motif. On the other hand, in order to decrease expression levels, the consensus sequence is disrupted, meaning that the sequence of the motif is altered in a way, which (further) decreases its similarity to the consensus sequence.

A disruption of a core promoter consensus sequence can be achieved by a point mutation, which transforms one nucleotide, which is in accordance with the consensus sequence at this position, into one, which is not in agreement with the consensus sequence at this position. A single point mutation resulting in a decreased expression level of the target gene is particularly advantageous, because it represents a minimal modification, which will likely not fall under regulations restricting the production and use of genetically modified organisms. Alternatively, one or more nucleotides of the sequence of a core promoter element can be deleted and thus altered to no longer contain conserved nucleotides of the corresponding consensus sequence. This results in a decrease of promoter activity and thus in lower expression levels of the target gene.

The method described above is broadly applicable to decrease the expression of any nucleic acid molecule of interest in a cellular context. The application is not limited to certain promoters or nucleic acid molecules of interest or combinations of both. The nucleic acid molecule of interest can be endogenous to the cell or organism that it is expressed in. In this case the promoter to be modified may be the promoter that natively controls the expression of this nucleic acid molecule of interest in the cell or organism but it is also possible that the endogenous nucleic acid molecule of interest is under the control of a heterologous promoter, which does not natively control its expression. Alternatively, the nucleic acid molecule of interest is exogenous to the cell or organism that it is expressed in. In this case the promoter to be modified may also be exogenous to the cell or organism but it may be the promoter that the nucleic acid molecule of interest is controlled by in its native cellular environment. On the other hand, the promoter to be modified may also be exogenous to the cell or organism in that it is to be modified and at the same time be heterologous to the nucleic acid molecule of interest.

In one embodiment of the method described above, the core promoter consensus sequence is a TATA box motif, a Y-patch motif, an initiator element, or a downstream promoter element and/or the at least one modification is a point mutation disrupting a TATA box motif and/or the at least one modification is a targeted deletion of one or more consecutive nucleotides comprising one or more nucleotides forming or being part of a TATA box motif in the core promoter sequence.

A TATA box motif can be found in many promoters within 100 nucleotides upstream of the transcription start site. In particular, a TATA box motif is found about 25 to 40 nt, preferably 31 to 32 nt, upstream of the transcription start site. The TATA box motif also represents the binding site for TBP (TATA box binding protein) and can therefore be identified by its interaction with the TBP. The consensus sequence for the TATA box motif in plants is given in SEQ ID NO: 11, wherein W stands for A or T. An ideal TATA box motif is represented by SEQ ID NO: 13. Not every TATA box motif exactly corresponds to the consensus sequence but can differ in one or more positions. However, even a different TATA box motif can still be identified by alignment and matching.

A Y-patch motif is commonly found in plant promoters and comprises a number of consecutive C and T nucleotides. A conserved consensus motif present in Y-patches is given in SEQ ID NO: 12, wherein Y stands for C or T. Y-patches may vary in length but they are usually also found within 100 nucleotides upstream of the transcription start site (position - 1 to -100 relative to TSS), preferably at a position between -10 to -60 relative to TSS.

An initiator element (Inr) is a core promoter sequence, which has a similar function as the TATA box and can also enable transcription initiation in the absence of a TATA box. It facilitates the binding of transcription factor II D, which is part of the RNA polymerase II preinitiation complex. The Inr encompasses the TSS and may contain a dimer motif (C/T A/G).

A downstream promoter element (DPE) is a core promoter sequence, which also plays a role in transcription initiation and is located downstream of the TSS. It is recognized by the transcription factor II D together with the Inr.

A disruption of a TATA box motif results in a sequence, which differs more from the consensus sequence defined above than it did before. A TATA box motif in a given promoter before the modification in step i) may correspond to the consensus sequence defined above or it may already differ from the consensus sequence in one or more positions. A point mutation disrupting a TATA box motif is a conversion of a nucleotide, which corresponds to the nucleotide at the same position in the consensus sequence, into a nucleotide, which differs from the nucleotide at the same position in the consensus sequence. Alternatively, the TATA box motif can be disrupted by deletion of one or more nucleotides, which form the TATA box motif or which are a part of the TATA box motif according to the consensus sequence. As demonstrated in the examples, a disruption of the TATA box consensus sequence by point mutation or deletion of nucleotides results in a decreased expression of the nucleic acid molecule of interest.

In another embodiment of the method described above, the at least one modification is a targeted deletion of one or more nucleotides comprising at least one, two, three, four, five, six, seven, eight or nine consecutive nucleotides forming or being part of a TATA box motif in the core promoter sequence and/or, the at least one modification is a targeted deletion of one or more nucleotides comprising at least one, two, three, four, five, six, seven, eight, nine, ten, eleven, twelve, thirteen, fourteen, fifteen, twenty, twenty-five, thirty, thirty-five, forty, forty-five, or at least fifty consecutive nucleotides of a Y-patch in the core promoter sequence.

A targeted deletion may affect one or more nucleotides of a core promoter consensus sequence. The deletion can affect nucleotides forming the TATA box motif or being part of the TATA box motif. It has also been demonstrated in the examples that a deletion of nucleotides being part of a Y-patch results in a decreased expression of the nucleic acid molecule of interest. As a Y-patch can vary in length, the number of nucleotides to be deleted can vary accordingly and impacts the decrease in expression as explained further below.

The present invention allows to adjust the expression of a target gene of interest to a desired level and avoid a complete knockout, which may have undesirable side effects. By selecting a targeted modification of the promoter sequence, a fine-tuning of the expression level is possible.

In one embodiment of the method described above, the expression level of the nucleic acid molecule of interest is decreased by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10% compared to the expression level of the nucleic acid molecule of interest under the control of an unmodified control promoter.

The reduction of expression is given in percentage of the expression of the nucleic acid molecule under the control of the unmodified control promoter, in particular the promoter present before the modification is introduced in step i) of the method described above.

It was found out in the context of the present invention, that the position at which the modification is introduced within a certain motive and also the amount of nucleotides affected by the modification has an impact on how large the decrease in expression is. In particular, it was observed that a point mutation affecting one or two nucleotide(s) at the 3'- or the 5'-end of the TATA box motif may only reduce the expression by about 50%, while point mutations in a more central region, i.e. at least one nucleotide apart from the 3'- or the 5'-end may result in a much larger decrease of around 80 or 90%. Deletion of two or more nucleotides from the TATA box motif results in a reduction of expression of around 90%. For the Y-patch motif it was observed that deletions of about half the nucleotides forming the Y-patch gave a decrease in expression of around 60 or 70%, while a deletion of most of the nucleotides forming the Y-patch reduced the expression by around 90%.

In one embodiment of the method described above, the
(a) the expression level of the nucleic acid molecule of interest is decreased by 40 to 80%, preferably 40 to 60%, compared to the expression level of the nucleic acid molecule of interest under the control of an unmodified control promoter when the at least one modification is a point mutation affecting the one or the two consecutive nucleotides located at the 5'-end and/or at the 3'-end of a TATA box motif or wherein the modification is a targeted deletion of consecutive nucleotides comprising 20 to 60%, preferably 30 to 50% of the nucleotides forming a Y-patch motif; or
(b) the expression level of the nucleic acid molecule of interest is decreased by 50 to 95%, preferably 70 to 95%, compared to the expression level of the nucleic acid molecule of interest under the control of an unmodified control promoter when the at least one modification is a point mutation affecting one or two consecutive nucleotides located at least one nucleotide apart from the 5'-end and/or the 3'-end of a TATA box motif, or wherein the modification is a targeted deletion of two or more nucleotides comprising at least two, at least three, at least four, at least five, at least six or at least seven nucleotides forming or being part of a TATA box motif, or wherein the modification is a targeted deletion of consecutive nucleotides comprising 75 to 95%, preferably 80 to 90% of the nucleotides forming a Y-patch motif.

The amount of decrease of expression when a certain nucleotide is mutated or deleted in a conserved motif, in particular the TATA box motif, also depends on the degree of conservation of that specific position of the motive. As can be inferred from Figure 5, certain positions in the TATA box motif are more conserved than others. If a less conserved position is mutated in a way that it differs more from the consensus sequence, the decrease in expression is less pronounced than when a highly conserved position is mutated in a way that it differs more from the consensus sequence.

As demonstrated in Example 4 and Figure 7, when point mutations are introduced into an ideal TATA box motif (SEQ ID NO: 13), which corresponds to the consensus sequence (SEQ ID NO: 11), mutations at the 5'- or 3'-end, i.e. at positions 1 or 9, result in a moderate reduction of promoter activity, while mutations in positions more in the centre, i.e. at positions 2, 6 or 8, have a more pronounced effect. The point mutations A6-T and T8-A result only a slight reduction of promoter activity because the nucleotides are exchanged for nucleotides, which are also frequently found in the respective positions.

In one embodiment of the method described above, the at least one modification is introduced by mutagenesis or by site-specific modification techniques using a site-specific nuclease or an active fragment thereof and/or a base editor.

Mutagenesis techniques can be based on chemical induction (e.g., EMS (ethyl methanesulfonate) or ENU (N-ethyl-N-nitrosourea)) or physical induction (e.g. irradiation with UV or gamma rays). In plant development, TILLING is well-known to introduce small modification like SNPs.

Site-specific modification may be achieved by introducing a site-specific nuclease or an active fragment thereof. Site-specific DNA cleaving activities of meganucleases, zinc finger nucleases (ZFNs), transcription activator-like effector nucleases (TALENs), or the clustered regularly interspaced short palindromic repeat (CRISPR), mainly the CRISPR/Cas9 technology have been widely applied in site-directed modifications of animal and plant genomes. The nucleases cause double strand breaks (DSBs) at specific cleaving sites, which are repaired by nonhomologous end-joining (NHEJ) or homologous recombination (HR). More recently discovered CRISPR systems include CRISPR/Cpf1, CRISPR/C2c2, CRISPR/CasX, CRISPR/CasY and CRISPR/Cmr, CRISPR/MAD7 or CRISPR/CasZ. Recombinases and Transposases catalyze the exchange or relocation of specific target sequences and can therefore also be used to create targeted modifications.

Furthermore, a base editing technique can be used to introduce a point mutation. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalysed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Gaudelli et al ((2017). Programmable base editing of A• T to G• C in genomic DNA without DNA cleavage. Nature, 551(7681), 464.) described adenine base editors (ABEs) that mediate the conversion of A•T to G•C in genomic DNA.

In one embodiment of the method described above, step (i) includes: introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

The introduction of the respective tool(s) in step i) may e.g. be achieved by means of transformation, transfection or transduction. Besides transformation methods based on biological approaches, like *Agrobacterium* transformation or viral vector mediated plant transformation, methods based on physical delivery methods, like particle bombardment or microinjection, have evolved as prominent techniques for importing genetic material into a plant cell or tissue of interest. Helenius et al. ("Gene delivery into intact plants using the HeliosTM Gene Gun", Plant Molecular Biology Reporter, 2000, 18 (3):287-288) discloses a particle bombardment as physical method for transferring material into a plant cell. Currently, there are a variety of plant transformation methods to introduce genetic material in the form of a genetic construct into a plant cell of interest, comprising biological and physical means known to the skilled person on the field of plant biotechnology and which can be applied. Notably, said delivery methods for transformation and transfection can be applied to introduce the required tools simultaneously. A common biological means is transformation with *Agrobacterium spp.* which has been used for decades for a variety of different plant materials. Viral vector mediated plant transformation represents a further strategy for introducing genetic material into a cell of interest. Physical means finding application in plant biology are particle bombardment, also named biolistic transfection or microparticle-mediated gene transfer, which refers to a physical delivery method for transferring a coated microparticle or nanoparticle comprising a nucleic acid or a genetic construct of interest into a target cell or tissue. Physical introduction means are suitable to introduce nucleic acids, i.e., RNA and/or DNA, and proteins. Likewise, specific transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising *inter alia* transfection with calcium phosphate, transfection using liposomes, e.g., cationic liposomes, or transfection with cationic polymers, including DEAD-dextran or polyethylenimine, or combinations thereof. Every delivery method has to be specifically fine-tuned and optimized so that a construct of interest can be introduced into a specific compartment of a target cell of interest in a fully functional and active way. The above delivery techniques, alone or in combination, can be used to introduce the necessary constructs, expression cassettes or vectors carrying the required tools i.e. a site-specific effector complex or at least one subcomponent thereof, i.e., at least one site-specific nuclease, at least one guide RNA, at least one repair template, or at least one base editor, or the sequences encoding the aforementioned subcomponents, according to the present invention into a target cell, *in vivo* or *in vitro.*

After its import, e.g. by transformation or transfection by biological or physical means, the nucleic acid construct or the expression cassette can either persist extrachromosomally, i.e. non integrated into the genome of the target cell, for example in the form of a double-stranded or single-stranded DNA, a double-stranded or single-stranded RNA. Alternatively, the construct, or parts thereof, according to the present disclosure can be stably integrated into the genome of a target cell, including the nuclear genome or further genetic elements of a target cell, including the genome of plastids like mitochondria or chloroplasts. A nucleic acid construct or an expression cassette may also be integrated into a vector for delivery into the target cell or organism.

The cell, in which the expression level of a nucleic acid molecule of interest is decreased in the method described above, maybe a plant, preferably wherein the plant originates from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, Spinacia or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Allium tuberosum, Helianthus annuus, Helianthus tuberosus* and/or *Spinacia oleracea.*

The nucleic acid molecule of interest, the expression of which is decreased in the method according to the invention, may be selected from susceptibility genes for pathogen resistance (e.g. CPL3 gene (EP 19 160408.1), MLO gene), developmental genes (e.g. CLV3 gene), genes involved in the regulation bolting and/or flowering of plants (AP1 and FUL (WO/2017/072304), VIL3 (EP 2 478 007 B1), genes affecting the quality of harvested products (cytochrome P450 flavonoid 3',5'-hydroxylase (F35H) (EP 18 169 122.1)), or genes conferring the ability of an haploid inducer (patatin-like phospholipase A (US 10,190,125 B2)).

In another aspect, the present invention provides a method for producing a cell or an organism, preferably a plant cell or a plant, having a decreased expression level of a nucleic acid molecule of interest, comprising the steps:
(i) providing a cell comprising the nucleic acid molecule of interest, which is expressed under the control of a promoter;
(ii) introducing at least one modification into the promoter sequence of the nucleic acid molecule of interest, wherein the modification is at least one, and preferably exactly one point mutation and/or a targeted deletion of one or more defined and consecutive nucleotides, wherein the modification disrupts at least one core promoter consensus sequence, and
(iii) obtaining a cell having a decreased expression level of the nucleic acid molecule of interest compared to the expression level of the nucleic acid molecule of interest under the control of an unmodified control promoter and optionally, culturing the cell to obtain an organism having a decreased expression level of a nucleic acid molecule of interest.

In one embodiment of the method for producing a cell or an organism described above, the core promoter consensus sequence is a TATA box motif, a Y-patch motif, an initiator element, or a downstream promoter element and/or the at least one modification is a point mutation disrupting a TATA box motif and/or the at least one modification is a targeted deletion of one or more consecutive nucleotides comprising one or more nucleotides forming or being part of a TATA box motif in the core promoter sequence.

In another embodiment of the method for producing a cell or an organism described above, the at least one modification is a targeted deletion of one or more nucleotides comprising at least one, two, three, four, five, six, seven, eight or nine consecutive nucleotides forming or being part of a TATA box motif in the core promoter sequence and/or the at least one modification is a targeted deletion of one or more nucleotides comprising at least one, two, three, four, five, six, seven, eight, nine, ten, eleven, twelve, thirteen, fourteen, fifteen, twenty, twenty-five, thirty, thirty-five, forty, forty-five, or at least fifty consecutive nucleotides of a Y-patch in the core promoter sequence.

In a further embodiment of the method for producing a cell or an organism described above, the expression level of the nucleic acid molecule of interest is decreased by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10% compared to the expression level of the nucleic acid molecule of interest under the control of an unmodified control promoter.

In one embodiment of the method for producing a cell or an organism described above,
(a) the expression level of the nucleic acid molecule of interest is decreased by 40 to 80%, preferably 40 to 60%, compared to the expression level of the nucleic acid molecule of interest under the control of an unmodified control promoter when the at least one modification is a point mutation affecting the one or the two consecutive nucleotides located at the 5'-end and/or at the 3'-end of a TATA box motif or wherein the modification is a targeted deletion of consecutive nucleotides comprising 20 to 60%, preferably 30 to 50% of the nucleotides forming a Y-patch motif; or
(b) the expression level of the nucleic acid molecule of interest is decreased by 50 to 95%, preferably 70 to 95%, compared to the expression level of the nucleic acid molecule of interest under the control of an unmodified control promoter when the at least one modification is a point mutation affecting one or two consecutive nucleotides located at least one nucleotide apart from the 5'-end and/or the 3'-end of a TATA box motif, or wherein the modification is a targeted deletion of two or more nucleotides comprising at least two, at least three, at least four, at least five, at least six or at least seven nucleotides forming or being part of a TATA box motif, or wherein the modification is a targeted deletion of consecutive nucleotides comprising 75 to 95%, preferably 80 to 90% of the nucleotides forming a Y-patch motif.

In another embodiment of the method for producing a cell or an organism described above, the at least one modification is introduced in step (ii) by mutagenesis or by site-specific modification techniques using a site-specific nuclease or an active fragment thereof and/or a base editor, preferably step (ii) includes: introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

The cell or organism obtained in the method for producing a cell or an organism described above may be a plant cell or a plant originating from a genus selected from the group consisting of *Hordeum, Sorghum, Saccharum, Zea, Setaria, Oryza, Triticum, Secale, Triticale, Malus, Brachypodium, Aegilops, Daucus, Beta, Eucalyptus, Nicotiana, Solanum, Coffea, Vitis, Erythrante, Genlisea, Cucumis, Marus, Arabidopsis, Crucihimalaya, Cardamine, Lepidium, Capsella, Olmarabidopsis, Arabis, Brassica, Eruca, Raphanus, Citrus, Jatropha, Populus, Medicago, Cicer, Cajanus, Phaseolus, Glycine, Gossypium, Astragalus, Lotus, Torenia, Allium, Spinacia or Helianthus,* preferably, the plant or plant cell originates from a species selected from the group consisting of *Hordeum vulgare, Hordeum bulbusom, Sorghum bicolor, Saccharum officinarium, Zea spp., including Zea mays, Setaria italica, Oryza minuta, Oryza sativa, Oryza australiensis, Oryza alta, Triticum aestivum, Triticum durum, Secale cereale,* Triticale, *Malus domestica, Brachypodium distachyon, Hordeum marinum, Aegilops tauschii, Daucus glochidiatus, Beta spp.,* including *Beta vulgaris, Daucus pusillus, Daucus muricatus, Daucus carota, Eucalyptus grandis, Nicotiana sylvestris, Nicotiana tomentosiformis, Nicotiana tabacum, Nicotiana benthamiana, Solanum lycopersicum, Solanum tuberosum, Coffea canephora, Vitis vinifera, Erythrante guttata, Genlisea aurea, Cucumis sativus, Marus notabilis, Arabidopsis arenosa, Arabidopsis lyrata, Arabidopsis thaliana, Crucihimalaya himalaica, Crucihimalaya wallichii, Cardamine nexuosa, Lepidium virginicum, Capsella bursa pastoris, Olmarabidopsis pumila, Arabis hirsute, Brassica napus, Brassica oleracea, Brassica rapa, Raphanus sativus, Brassica juncacea, Brassica nigra, Eruca vesicaria subsp. sativa, Citrus sinensis, Jatropha curcas, Populus trichocarpa, Medicago truncatula, Cicer yamashitae, Cicer bijugum, Cicer arietinum, Cicer reticulatum, Cicer judaicum, Cajanus cajanifolius, Cajanus scarabaeoides, Phaseolus vulgaris, Glycine max, Gossypium sp., Astragalus sinicus, Lotus japonicas, Torenia fournieri, Allium cepa, Allium fistulosum, Allium sativum, Allium tuberosum, Helianthus annuus, Helianthus tuberosus* and/or *Spinacia oleracea.* **[Herr Dr. Westhoff, Frau Dr. Streitner: Diese Liste haben wir aus der Anmeldung zu den "promoter activating elements" entnommen. Bitte bei Bedarf anpassen, danke!]**

The nucleic acid molecule of interest, the expression of which is decreased in the cell or organism obtained by the method described above, may be selected from susceptibility genes for pathogen resistance (e.g. CPL3 gene (EP 19 160 408.1), MLO gene), developmental genes (e.g. CLV3 gene), genes involved in the regulation bolting and/or flowering of plants (AP1 and FUL (WO/2017/072304), VIL3 (EP 2 478 007 B1), genes affecting the quality of harvested products (cytochrome P450 flavonoid 3',5'-hydroxylase (F35H) (EP 18 169 122.1)), or genes conferring the ability of an haploid inducer (patatin-like phospholipase A (US 10,190,125 B2)).

In a further aspect, the invention relates to a cell obtained or obtainable by a method according to any of the embodiments of the method for producing a cell or an organism described above.

In yet a further aspect, the invention also relates to an organism or a part thereof obtained or obtainable by culturing a cell according to the aspect above.

The organism, in particular a plant, obtained by the method described above, is preferably a non-genetically modified organism carrying only a minimal modification in its genome, i.e. a single point mutation or a deletion of one or more nucleotides. In order to obtain such an organism, any of the tools used to introduce the modification may be expressed transiently in the cell, so that they are no longer present after culturing.

### Example 1: Repression of promoter activity by introduction of point mutations in the TATA-box

Figure 1 shows a schematic overview of the structure of the corn CPL3 promoter (SEQ ID NO: 1). Point mutations are introduced in the promoter of interest (e.g. ZmCPL3) within the region of the TATA-box (Figure 2A). The effect on promoter activity is measured in a transient assay system based on leaf bombardment (Figure 2B) or callus bombardment (Figure 2C) with respective promoter-reporter constructs followed by luciferase measurement. These point mutations result in a severe decrease of promoter activity, especially when very conserved nucleotides of the TATA-box motif are targeted, like in the promoter variants CPL3v3 (SEQ ID NO: 2) and CPL3v4 (SEQ ID NO: 3). If the point mutations are placed in the less conserved nucleotides of the TATA-box motif, like in the promoter variants CPL3v5 (SEQ ID NO: 4) and CPL3v6 (SEQ ID NO: 5), the effect is a more moderate decrease in promoter activity. This allows for a precise modulation of the desired gene expression, e.g. to avoid pleiotropic negative effects by complete, or almost complete loss of gene function. The promoter variant CPL3v7 (SEQ ID NO: 6) provides an internal control by modifying the TATA-box in the direction of being closer to the conserved sequence of the motif and thereby increasing the promoter activity. The promoter variant CPL3v8 (SEQ ID NO: 7 and 8) is an example for a point mutation introduced outside the TATA-box region having no effect on the promoter activity.

### Example 2: Repression of promoter activity by small deletions in the TATA-box

Figure 1 shows a schematic overview of the structure of the corn CPL3 promoter. Small deletions are introduced in the promoter of interest (e.g. ZmCPL3) within the region of the TATA-box (Figure 3A). The effect on promoter activity is measured in a transient assay system based on leaf bombardment (Figure 3B) or callus bombardment (Figure 3C) with respective promoter-reporter constructs followed by luciferase measurement. These small deletions within the TATA-box motif result in a severe decrease of promoter activity, like it is visible for the promoter variants CPL3v9, CPL3v10 and CPL3v11.

### Example 3: Repression of promoter activity by deletions in the Y-patch

Figure 1 shows a schematic overview of the structure of the corn CPL3 promoter. Deletions are introduced in the promoter of interest (e.g. ZmCPL3) within the region of the Y-patch motif (SEQ ID NO: 10) (Figure 4A). The effect on promoter activity is measured in a transient assay system based on leaf bombardment (Figure 4B) or callus bombardment (Figure 4C) with respective promoter-reporter constructs followed by luciferase measurement. Deletions within the Y-patch motif result in a decrease of promoter activity, like it is visible for the promoter variants CPL3v12 and CPL3v13. The effect is more moderate compared to small deletions placed directly in the region of the TATA-box motif like described in example 2.

### Example 4: Repression of promoter activity by placing point mutations into highly conserved or moderately conserved positions of the TATA-box consensus motif

Figure 7A gives an overview about the point mutations which have been introduced into the perfect TATA-box motif (SEQ ID NO: 13) present in three different corn promotors (7B-D) and one sugar beet promoter (7E). The effect of these point mutations on promoter activity is measured in a transient assay system based on leaf bombardment with respective promoter-reporter constructs followed by luciferase measurement. Mutations at position 1 (C1-A (SEQ ID NO: 14), C1-T (SEQ ID NO: 15), C1-G (SEQ ID NO: 16)) and at position 9 (A9-G (SEQ ID NO: 25), A9-C (SEQ ID NO: 26), A9-T (SEQ ID NO: 27)) result in a moderate reduction of promoter activity. The repression of promoter activity is more pronounced when point mutations are done to nucleotides in the center of the TATA-box consensus motif (T2-C and T2-A in combination with C1-T or A6-C, A6-G, T8-C and T8-G). The two point mutations A6-T (SEQ ID NO: 19) and T8-A (SEQ ID NO: 22) show a moderate effect, if an effect at all, as in these cases the nucleotide is exchanged for the nucleotide which is also described to be very likely present at this position in a TATA-box motif (see Figure 5A and 5B).

## Claims

1. A method for gradually decreasing the expression level of a nucleic acid molecule of interest in at least one cell, preferably a plant cell, comprising the following steps:
(i) introducing at least one modification into a promoter sequence of the nucleic acid molecule of interest, wherein the modification is at least one, and preferably exactly one, point mutation and/or a targeted deletion of one or more defined and consecutive nucleotides, wherein the modification disrupts at least one core promoter consensus sequence; and
(ii) obtaining at least one cell showing a decreased expression level of a nucleic acid molecule of interest compared to the expression level of the nucleic acid molecule of interest under the control of an unmodified control promoter.

2. The method of claim 1, wherein the core promoter consensus sequence is a TATA box motif, a Y-patch motif, an initiator element, or a downstream promoter element and/or wherein the at least one modification is a point mutation disrupting a TATA box motif and/or wherein the at least one modification is a targeted deletion of one or more consecutive nucleotides comprising one or more nucleotides forming or being part of a TATA box motif in the core promoter sequence.

3. The method of claim 1 or 2, wherein the at least one modification is a targeted deletion of one or more nucleotides comprising at least one, two, three, four, five, six, seven, eight or nine consecutive nucleotides forming or being part of a TATA box motif in the core promoter sequence and/or, wherein the at least one modification is a targeted deletion of one or more nucleotides comprising at least one, two, three, four, five, six, seven, eight, nine, ten, eleven, twelve, thirteen, fourteen, fifteen, twenty, twenty-five, thirty, thirty-five, forty, forty-five, or at least fifty consecutive nucleotides of a Y-patch in the core promoter sequence.

4. The method of any of the preceding claims, wherein the expression level of the nucleic acid molecule of interest is decreased by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10% compared to the expression level of the nucleic acid molecule of interest under the control of an unmodified control promoter.

5. The method of any of the preceding claims, wherein
(a) the expression level of the nucleic acid molecule of interest is decreased by 40 to 80%, preferably 40 to 60% , compared to the expression level of the nucleic acid molecule of interest under the control of an unmodified control promoter when the at least one modification is a point mutation affecting the one or the two consecutive nucleotides located at the 5'-end and/or at the 3'-end of a TATA box motif or wherein the modification is a targeted deletion of consecutive nucleotides comprising 20 to 60%, preferably 30 to 50% of the nucleotides forming a Y-patch motif; or wherein
(b) the expression level of the nucleic acid molecule of interest is decreased by 50 to 95%, preferably 70 to 95% compared to the expression level of the nucleic acid molecule of interest under the control of an unmodified control promoter when the at least one modification is a point mutation affecting one or two consecutive nucleotides located at least one nucleotide apart from the 5'-end and/or the 3'-end of a TATA box motif, or wherein the modification is a targeted deletion of two or more nucleotides comprising at least two, at least three, at least four, at least five, at least six or at least seven nucleotides forming or being part of a TATA box motif, or wherein the modification is a targeted deletion of consecutive nucleotides comprising 75 to 95%, preferably 80 to 90% of the nucleotides forming a Y-patch motif.

6. The method of any of the preceding claims, wherein the at least one modification is introduced by mutagenesis or by site-specific modification techniques using a site-specific nuclease or an active fragment thereof and/or a base editor.

7. The method of any of the preceding claims, wherein step (i) includes: introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

8. A method for producing a cell or an organism, preferably a plant cell or a plant, having a decreased expression level of a nucleic acid molecule of interest, comprising the steps:
(i) providing a cell comprising the nucleic acid molecule of interest, which is expressed under the control of a promoter;
(ii) introducing at least one modification into the promoter sequence of the nucleic acid molecule of interest, wherein the modification is at least one, and preferably exactly one point mutation and/or a targeted deletion of one or more defined and consecutive nucleotides, wherein the modification disrupts at least one core promoter consensus sequence, and
(iii) obtaining a cell having a decreased expression level of the nucleic acid molecule of interest compared to the expression level of the nucleic acid molecule of interest under the control of an unmodified control promoter and optionally, culturing the cell to obtain an organism having a decreased expression level of a nucleic acid molecule of interest.

9. The method of claim 8, wherein the core promoter consensus sequence is a TATA box motif, a Y-patch motif, an initiator element, or a downstream promoter element and/or wherein the at least one modification is a point mutation disrupting a TATA box motif and/or wherein the at least one modification is a targeted deletion of one or more consecutive nucleotides comprising one or more nucleotides forming or being part of a TATA box motif in the core promoter sequence.

10. The method of claim 8 or 9, wherein the at least one modification is a targeted deletion of one or more nucleotides comprising at least one, two, three, four, five, six, seven, eight or nine consecutive nucleotides forming or being part of a TATA box motif in the core promoter sequence and/or, wherein the at least one modification is a targeted deletion of one or more nucleotides comprising at least one, two, three, four, five, six, seven, eight, nine, ten, eleven, twelve, thirteen, fourteen, fifteen, twenty, twenty-five, thirty, thirty-five, forty, forty-five, or at least fifty consecutive nucleotides of a Y-patch in the core promoter sequence.

11. The method of any one of claims 8 to 10, wherein the expression level of the nucleic acid molecule of interest is decreased by up to 95%, up to 90%, up to 85%, up to 80%, up to 75%, up to 70%, up to 65%, up to 60%, up to 55%, up to 50%, up to 45%, up to 40%, up to 35%, up to 30%, up to 25%, up to 20%, up to 15%, up to 10% compared to the expression level of the nucleic acid molecule of interest under the control of an unmodified control promoter.

12. The method of any one of claims 8 to 11, wherein
(a) the expression level of the nucleic acid molecule of interest is decreased by 40 to 80%, preferably 40 to 60%, compared to the expression level of the nucleic acid molecule of interest under the control of an unmodified control promoter when the at least one modification is a point mutation affecting the one or the two consecutive nucleotides located at the 5'-end and/or at the 3'-end of a TATA box motif or wherein the modification is a targeted deletion of consecutive nucleotides comprising 20 to 60%, preferably 30 to 50% of the nucleotides forming a Y-patch motif; or wherein
(b) the expression level of the nucleic acid molecule of interest is decreased by 50 to 95%, preferably 75 to 95%, compared to the expression level of the nucleic acid molecule of interest under the control of an unmodified control promoter when the at least one modification is a point mutation affecting one or two consecutive nucleotides located at least one nucleotide apart from the 5'-end and/or the 3'-end of a TATA box motif, or wherein the modification is a targeted deletion of two or more nucleotides comprising at least two, at least three, at least four, at least five, at least six or at least seven nucleotides forming or being part of a TATA box motif, or wherein the modification is a targeted deletion of consecutive nucleotides comprising 75 to 95%, preferably 80 to 90% of the nucleotides forming a Y-patch motif.

13. The method of any of claims 8 to 12, wherein the at least one modification is introduced in step (ii) by mutagenesis or by site-specific modification techniques using a site-specific nuclease or an active fragment thereof and/or a base editor, preferably wherein step (ii) includes: introducing into the cell a site-specific nuclease or an active fragment thereof, or providing the sequence encoding the same, the site-specific nuclease inducing a single- or double-strand break at a predetermined location, preferably wherein the site-specific nuclease or the active fragment thereof comprises a zinc-finger nuclease, a transcription activator-like effector nuclease, a CRISPR/Cas system, including a CRISPR/Cas9 system, a CRISPR/Cpf1 system, a CRISPR/C2C2 system a CRISPR/CasX system, a CRISPR/CasY system, a CRISPR/Cmr system, a CRISPR/MAD7 system, a CRISPR/CasZ system, an engineered homing endonuclease, a recombinase, a transposase and a meganuclease, and/or any combination, variant, or catalytically active fragment thereof; and optionally when the site-specific nuclease or the active fragment thereof is a CRISPR nuclease: providing at least one guide RNA or at least one guide RNA system, or a nucleic acid encoding the same; and optionally providing at least one repair template nucleic acid sequence.

14. A cell obtained or obtainable by a method according to any of claims 8 to 13.

15. An organism or a part thereof, preferably a plant or part of a plant obtained or obtainable by culturing a cell of claim 14.
